(19) Europäisches Patentamt
European Patent Office
Office européen des brevets

(11) Veröffentlichungsnummer: 0 098 793
B1

# (12) EUROPÄISCHE PATENTSCHRIFT

(45) Veröffentlichungstag der Patentschrift: **25.01.89**

(51) Int. Cl.⁴: **C 07 C 177/00,** C 07 C 103/737, A 61 K 31/557

(21) Anmeldenummer: **83730065.6**

(22) Anmeldetag: **01.07.83**

(54) **Neue Carbacyclinamide, Verfahren zu ihrer Herstellung und ihre Verwendung als Arzneimittel.**

(30) Priorität: **02.07.82 DE 3225287**

(43) Veröffentlichungstag der Anmeldung: **18.01.84 Patentblatt 84/3**

(45) Bekanntmachung des Hinweises auf die Patenterteilung: **25.01.89 Patentblatt 89/4**

(84) Benannte Vertragsstaaten: **AT BE CH DE FR GB IT LI LU NL SE**

(56) Entgegenhaltungen:
**EP-A-0 011 591**
**EP-A-0 055 208**
**FR-A-2 422 634**
**GB-A-2 014 143**
**GB-A-2 070 596**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Radüchel, Bernd, Dr., Gollanczstrasse 132, D-1000 Berlin 28 (DE)**
Erfinder: **Vorbrüggen, Helmut, Prof. Dr., Wilkestrasse 7, D-1000 Berlin 27 (DE)**
Erfinder: **Mannesmann, Gerda, Dr., Sachsenring 22/24, D-5000 Köln 1 (DE)**
Erfinder: **Nieuweboer, Bob, Bärbelweg 16, D-1000 Berlin 27 (DE)**
Erfinder: **Town, Michael Harold, Dr., Buchsbaumweg 3, D-1000 Berlin 47 (DE)**

**EP 0 098 793 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue Carbacyclinamide, Verfahren zu ihrer Herstellung sowie ihre Verwendung als Arzneimittel.

In den deutschen Offenlegungsschriften DE-OS-2 845 770, 2 900 352, 2 902 442, 2 904 655, 2 909 088, 3 209 702, 3 204 443, 3 048 906 und 2 912 409 werden (5E)- und (5Z)-6a-Carbaprostaglandin-$I_2$-Analoga beschrieben. In der deutschen Offenlegungsschrift DE-OS-3 105 588 werden Carbacyclin-(hydroxyalkylamide) beansprucht, die jedoch in der $C_{15}$-Alkylkette keine ungesättige Alkyl-Gruppen tragen. Das der DE-OS-2 845 770 entsprechende EP-Patent 11 591 beschreibt das als Blutdruck-senkenden Wirkstoff bekannte Iloprost. Die Nomenklatur der erfindungsgemäßen Verbindungen basiert auf einem Vorschlag von Morton und Brokaw (J. Org. Chem. <u>44</u>, 2880 [1979]. Bei der Synthese dieser Verbindungen entstehen stets zwei Doppelbindungsisomere, die durch den Zusatz (5E) oder (5Z) charakterisiert werden. Die beiden Isomeren dieses Prototyps werden durch folgende Strukturformel verdeutlicht:

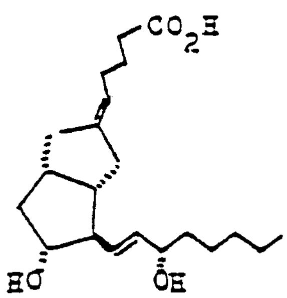

(5E)-6a-Carbaprostaglandin-$I_2$

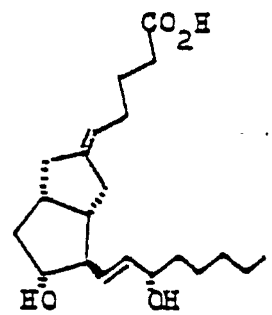

(5Z)-6a-Carbaprostaglandin-$I_2$

Aus dem sehr umfangreichen Stand der Technik der Prostacycline und ihrer Analoga weiß man, daß diese Stoffklasse augrund ihrer biologischen und pharmakologischen Eigenschaften zur Behandlung von Säugetieren, einschließlich Menschen, geeignet ist. Ihre Verwendung als Arzneimittel stößt jedoch häufig auf Schwierigkeiten, da sie eine für therapeutische Zwecke zu kurze Wirkungsdauer besitzen. Alle Struckturveränderungen haben das Ziel, die Wirkungsdauer sowie die Selektivität der Wirksamkeit zu steigern.

Es wurde nun gefunden, daß (Hydroxyalkyl)-Amide von in 17-19-stellung ungesättigten Carbacyclinen eine deutlich längere Wirkung besitzen als die freien Säuren der Carbacyclin-Derivate. Die erfindungsgemäßen Verbindungen wirken bronchodilatatorisch und sind zur Inhibierung der Thrombozytenaggregation, zur Blutdrucksenkung über eine Vasodilation und zur Hemmung der Magensäuresekretion geeignet.

Die Erfindung betrifft Carbacyclinamide der Formel I

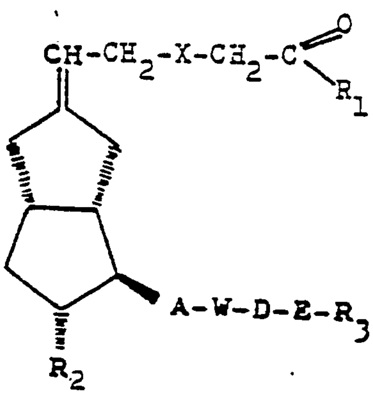

(I),

worin

- $R_1$ die Reste $NHR_4$ mit $R_4$ als niederer Mono- oder Polyhydroxyalkylgruppe mit 2 - 8 C-Atomen,
- $R_2$ eine freie oder funktionell abgewandelte Hydroxygruppe,
- $R_3$ eine Alkyl mit 1 - 7 C-Atomen,
- X die Gruppe $-CH_2-$,
- A eine trans-CH=CH- oder -C≡C-Gruppe,
- W eine Hydroxymethylengruppe oder eine 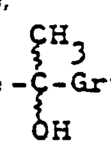 -Gruppe ,

  wobei die OH-Gruppe α- oder β-ständig sein kann,

D die Gruppe 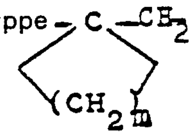 mit m gleich 1 - 3, eine geradkettige Alkylengruppe mit 1 - 5 C-Atomen oder eine verzweigte Alkylengruppe mit 2 - 5 C-Atomen,

E eine -C≡C-Gruppe oder eine $-CR_6=CR_7$-Gruppe, wobei $R_6$ und $R_7$ ein Wasserstoffatom oder eine Alkylgruppe mit 1 - 5 C-Atomen bedeuten, darstellen.

Die Verbindungen der Formel I stellen sowohl (5E)- als auch (5Z)-Isomere dar.

Die Reste $R_4$ enthalten als gerad- oder verzweigtkettige niedere Mono- oder Polyhydroxyalkylreste 2 - 8 Kohlenstoffatome, vorzugsweise 2 - 5 Kohlenstoffatome. Geradkettige Reste von $R_4$ bestehen vorzugsweise aus 2 - 4 Kohlenstoffatomen, verzweigtkettige vorzugsweise aus 3 - 5 Kohlenstoffatomen. Die Hydroxgruppen in den Resten $R_4$ können als primäre oder sekundäre Hydroxygruppen vorliegen. Die Reste $R_4$ können 1 - 5 Hydroxygruppen enthalten, bevorzugt sind 1 - 3 Hydroxygruppen. Als Reste $R_4$ seien beispielsweise genannt: 2-Hydroäthyl-, 2-Hydropropyl-, 3-Hydroxypropyl-, 2-Hydroxy-1-methylpropyl-, 3-Hydroxy-1-methylpropyl-, 1-(Hydroxymethyl)-äthyl, 2-Hydroxybutyl-, 3-Hydroxybutyl-, 4-Hydroxybutyl-, 2-Hydroxy-1-methylbutyl-, 3-Hydroxy-1-methylbutyl, 4.Hydroxy-1-methylbutyl-, 3-Hydroxy-2-methylbutyl-, 4-Hydroxy-2-methylbutyl, 2-Hydroxyisobutyl-, 3-Hydroxy-isobutyl-, 2-Hydroxy-1,1-dimethyläthyl-, 3-Hydroxy-1,1-dimetylpropyl-, 2,3-Dihydroxypropyl-, 2,3-Dihydroxybutyl-, 2,4-Dihydroxybutyl-, 3,4-Dihydroxybutyl-, 3-Hydroxy-2-(hydroxymethyl)-propyl-, 2,3-Dihydroxy-1-methylpropyl-, 2-Hydroxy-3-(hydroxymethyl)-butyl-, 2,3,4-Trihydroxybutyl-, 2,4-Dihydroxy-3-(hydroxymetyl)-butyl-, 3-Hydroxy-2,2-bis (hydroxymethyl)-propyl-, 4-Hydroxy-3,3-bis(hydroxymethyl)-butyl, 4-Hydroxy-2,2-bis(hydroxymethyl)butyl-, 2-Hydroxy-1,1-bis(hydroxymethyl)-äthyl-, Glucosaminrest.

Als Alkylgruppen $R_3$ kommen gerad- und verzweigtkettige, gesättigte Alkylreste mit 1 - 7 C-Atomen i Frage. Beispielsweise genannt seien Methyl-, Äthyl-, Propyl-, Butyl-, Isobutyl-, tert-.Butyl-, Pentyl-, Hexyl-, Heptyl-.

Als Alkylengruppe D kommen geradkettige mit 1 - 5 C-Atomen oder verzweigtkettige Alkylenreste mit 2 - 5 C-Atomen in Frage. Beispielsweise seien genannt: Methylen, Äthylen, 1.2-Propylen, Äthyläthylen, Trimethylen, Tetramethylen, Pentamethylen, 1-Methyltetramethylen, 1-Methyltrimethylen, 1,1-Trimethylenäthylen.

Besonders bevorzugte Verbindungen dieser Erfindung sind solche mit E als -C≡C- oder $CR_6=CR_7$, worin beide Reste $R_6$ und $R_7$ eine Alkylgruppe mit 1 - 5 C-Atomen darstellen, mit einer 16-Methyl-Substitution und einer Mono- oder Polyhydroxyalkylamid-Gruppierung in 1-Stellung.

Die Erfindung betrifft ferner ein Verfahren zur Herstellung von Carbacyclinamiden der Formel I, dadurch gekennzeichnet, daß man eine Verbindung der Formel II

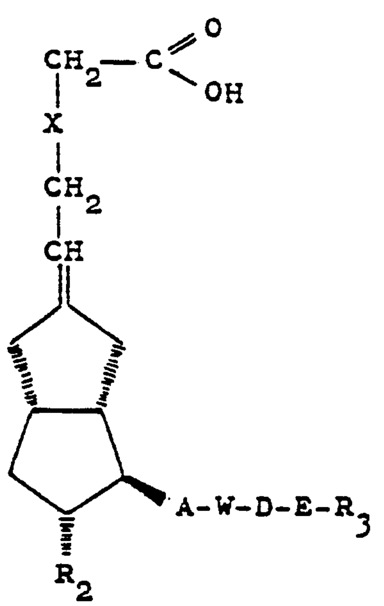

(II),

worin

$R_2$, $R_3$, X, A, W, D und E die oben angegebenen Bedeutungen aufweisen, gegebenenfalls nach Schutz anwesender freier Hydroxygruppen mit Aminen der Formel III

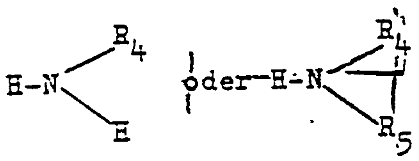

(III),

worin $R_4$ die oben angegebenen Bedeutungen aufweist, nach vorheriger Umsetzung mit Chlorameisensäureisobutylester in Gegenwart eines tertiären Amins umsetzt und gegebenenfalls anschließend geschützte Hydroxygruppen freisetzt und freie Hydroxygruppen verestert oder veräthert und in die Isomeren trennt.

Die Umsetzung der Verbindung der allgemeinen Formel II mit einem Amin der allgemeinen Formel III wird bei Temperaturen von -60°C bis 60°C, vorzugsweise 10°C bis 40°C, in einem inerten Lösungsmittel, beispielsweise Aceton, Acetonitril, Dimethylacetamid, Dimethylformamid oder Dimethylsulfoxid, vorgenommen.

Als Basen zur Herstellung der intermediären Anhydride kommen die dem Fachmann für derartige Amidierungen bekannten Basen, vorzugsweise tertiäre Basen, in Frage, beispielsweise Triäthylamin, Trimethylamin, Tributylamin, Trioctylamin, Pyridin, N,N-Diäthylisopropylamin.

Die Verbindungen der Formel II sind durch die eingangs erwähnten deutschen Offenlegungsschriften bekannt. Die Verbindungen der Formel III sind literaturbekannt.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normalerweise bei 0°C - 30°C nach 15 - 30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u. a., umsetzt.

Die Freisetzung einer funktionell abgewandelten OH-Gruppe zu den Verbindungen der allgemeinen Formell I erfolgt nach bekannten Methoden. Beispielsweise wird die Abspaltung von Ätherschutzgruppen in einer wässrigen Lösung einer organischen Säure, wie zum Beispiel Essigsäure, Propionsäure u. a. oder in einer wässrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkali- oder Erdalkali-carbonaten oder -hydroxyden in einem Alkohol oder der wässrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei -10°C bis 70°C, vorzugsweise bei 25°C.

Die Verbindungen dieser Erfindung wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoffe dar. Darüberhinaus weisen sie bei ähnlichem Wirkungsspektrum, verglichen mit entsprechenden Prostacyclinen, eine höhere Spezifität und vor allem eine wesentlich längere Wirksamkeit auf. Im Vergleich zu $PGI_2$ zeichnen sie sich durch größere Stabilität aus. Die hohe Gewebespezifität der neuen Carbacyclinamide zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarktes, peripherer Arterienerkrankungen, Arteriosklerose und Trombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen und Darmschleimhaut, Senkung des pulmonaren vaskulären Widerstandes und des pulmonaren Blutdruckes, Förderung der Nierendurchblutung, Anwendung an Stelle von Heparin oder als Adjuvans bei der Dialyse der Hämofiltration, Konservierung von Blutplasmakonserven, besonders von Blutplättchenkonserven, Erhöhung der zerebralen Durchblutung etc. Die Carbacycline dieser

Erfindung können auch in Kombination, z. B. mit β-Blockern oder Diuretika, verwendet werden.

Die Dosis der Verbindungen ist 1 - 1500 µg/kg/Tag, wenn sie am menschlichen Patienten verabreicht werden. Die Einheitsdosis für den pharmazeutischen akzeptablen Träger beträgt 0,01 - 100 mg.

Nach systemischer Applikation zeigen die neuen Carbacyclinamine z. B. gegenüber den freien Säuren eine deutlich verlängerte Wirkung.

Die erfindungsgemäßlen Wirkstoffe sollen in Verbindung mit den in der Galenik bekannten und üblichen Hilfsstoffen, z. B. zur Herstellung von Blutdrucksenkern, dienen. Die Erfindung betrifft damit auch Arzneimittel auf Basis der Verbindungen der Formel I und üblicher Hilfs- und Trägerstoffe.

Die nachfolgenden Beispiele sollen das erfindungsgemäße Verfahren erläutern.

**Beispiel 1**

(5E)-(16RS)-16-Methyl-18,18,19,19-tetradehydro-6a-carbaprostaglandin-$I_2$(2,3-dihydroxy-propyl)-amid

333 mg (SE)-(16RS)-16-Methyl-18,18,19,19-tetradehydro-6a-carbaprostaglandin-$I_2$-1,15-bis-tetrahydropyranyläther werden in 4 ml Aceton gelöst und bei 0°C mit 74 mg Triäthylamin und 100 mg Chlorameisensäureisobutylester versetzt. Man rührt 20 Minuten und fügt dann eine Lösung von 574 mg 1-Amino-2-3-dihydroxypropan in 9 ml Aceton und 9 ml Acetonitril zu und rührt 1 Stunde bei 24°C. Anschließend engt man im Vakuum ein, nimmt den Rückstand in 100 ml Methylenchlorid auf, schüttelt dreimal mit Sole, trocknet über Magnesiumsulfat und dampft im Vakuum ein.

Den Rückstand rührt man 18 Stunden bei 24°C mit 10 ml einer Mischung aus Essigsäure, Wasser, Tetrahydrofuran (65 : 35 : 10). Man dampft im Vakuum ein und reinigt den Rückstand durch Säulenchromatographie an Kieselgel. Mit Methylenchlorid/Isopropanol (8 + 2) erhält man 205 mg der Titelverbindung als farbloses Öl.

IR ($CHCl_3$): 3660, 3380, 2925, 1640, 970/cm

**Beispiel 2**

(5E)-(16RS)-16-Metyl-18,18,19,19,-tetradehydro-6a-carbaprostaglandin-$I_2$-(2-hydroxy-äthyl)-amid

Analog Beispiel 1 aus 500 mg (5E)-(16RS)-16-Methyl-18,18,19,19-tetradehydro-6a-carbaprostaglandin-$I_2$-11,15-bis-tetrahydropyranyläther in 4,5 ml Aceton, Triäthylamin und Chlorameisensäureisobutylester. Dazu wird eine Lösung von 577,2 mg 2-Aminoäthanol in 13,5 ml Aceton und 13,5 ml Acetonitril getropft. Ausbeute 586 mg, von den 300 mg weiterverarbeitet werden. Die Abspaltung der Tetrahydropyranyl-Schutzgruppen erfolgt analog Beispiel 1. Ausbeute 122 mg der Titelverbindung als Öl.

IR ($CHCl_3$): 3600, 3350 (breit), 2930, 1658, 1515, 968/cm

**Beispiel 3**

(5E)-(16RS)-16-Methyl-18,18,19,19-tetradehydro-6a-carbaprostaglandin-$I_2$-(2,3-4-trihydroxy-butyl)-amid

Analog Beispiel 1 aus (5E)-(16RS)-16-Methyl-18,18,19,19-tetradehydro-6a-carbaprostaglandin-$I_2$-11,15-bis-tetrahydropyranyl-äther und 2,3,4-Trihydroxybutyl-amin hergestellt.

IR ($CHCl_3$): 3600, 3380 (breit), 2930, 1670, 1585, 970/cm

**Beispiel 4**

(5E)-(16RS)-16-Methyl-18,18,19,19-tetradehydro-6a-carbaprostaglandin-$I_2$-glucosamid

Analog Beispiel 1 aus (5E)-(16RS)-16-Metyl-18,18,19,19-tetradehydro-6a-carbaprostaglandin-$I_2$-11,15-bis-tetrahydropyranyläther und Glucosamin hergestellt.

IR ($CHCl_3$): 3600, 3400 (breit), 2925, 1680, 1585, 965/cm

**Beispiel 5**

(5E)-(16RS)-13,14-Didehydro-16-methyl-18,18,19,19-tetradehydro-6a-carbaprostaglandin-$I_2$-(2,3-dihydroxypropyl)-amid

Analog Beispiel 1 aus (5E)-(16RS)-13,14-Didehydro-16-methyl-18,18, 19,19-tetradehydro-6a-carbaprostaglandin-$I_2$-11,15-bis-tetrahydropyranyläther und 1-Amino-2,3-dihydroxypropan.

IR ($CHCl_3$): 3600, 3390 (breit), 2925, 1680, 1575, 965/cm

**Beispiel 6**

(5E)-18,18,19,19-Tetradehydro-16,16-trimethylen-6a-carbaprostaglandin-$I_2$-(2,3-dihydroxypropyl)-amid

Analog Beispiel 1 aus (5E)-18,18,19,19-Tetradehydro-16,16-trimethylen-6a-carbaprostaglandin-$I_2$-11,15-bis-tetrahydropyranyläther und 1-Amino-2,3-dihydroxypropan.

IR ($CHCl_3$): 3600, 3410 (breit), 2930, 1690, 1585, 975/cm

**Beispiel 7**

(5E)-16,16-Dimethyl-18,18,19,19-tetradehydro-6a-carbaprostaglandin-$I_2$-(2,3-dihydroxypropyl)-amid

Analog Beispiel 1 aus (5E)-16,16-Dimethyl-18,18,19,19-tetradehydro-6a-carbaprostaglandin-$I_2$-11,15-bis-tetrahydropyranyläther und 1-Amino-2,3-dihydroxypropan.

IR ($CHCl_3$): 3600, 3400 (br.), 2930, 1690, 1580, 965/cm

**Beispiel 8**

(5E)-(16RS)-16,20-Dimethyl-18,18,19,19-tetradehydro-6a-carbaprostaglandin-$I_2$-(2,3-dihydroxypropyl)-amid

Analog Beispiel 1 aus (5E)-(16RS)-16,20-Dimethyl-18,18,19,19-tetradehydro-6a-carbaprostaglandin-$I_2$-11,15-bis-tetrahydropyranyläther und 1-Amino-2,3-dihydroxypropan.

IR ($CHCl_3$): 3600, 3390 (br.), 2925, 1680, 1575, 970/cm

**Beispiel 9**

(5E)-20-Methyl-18,18,19,19-tetradehydro-16,16-trimethylen-6a-carbaprostaglandin-$I_2$-(2,3-dihydroxypropyl)-amid

Analog Beispiel 1 aus (5E)-20-Methyl-18,18,19,19-tetradehydro-16,16-trimethylen-6a-carbaprostaglandin-$I_2$-11,15-bis-tetrahydropyranyläther und 1-Amino-2,3-dihydroxypropan.

IR ($CHCl_3$): 3600, 3410 (br.), 2930, 1685, 1570, 965/cm

**Beispiel 10**

(5E)-13,14-Didehydro-20-methyl-18,18,19,19-tetradehydro-16,16-trimethylen-6a-carbaprostaglandin-$I_2$-(2,3-dihydroxypropyl)-amid

Analog Beispiel 1 aus (5E)-13,14-Didehydro-20-methyl-18,18,19,19-tetradehydro-16,16-trimethylen-6a-carbaprostaglandin-$I_2$-11,15-bis-tetrahydropyranyläther und 1-Amino-2,3-dihydroxypropan.

IR ($CHCl_3$): 3600, 3410 (br.), 2920, 1680, 1585, 975/cm

**Beispiel 11**

(5E)-(16RS)-16,19-Dimethyl-18,19-didehydro-6a-carbaprostaglandin-$I_2$-(2,3-dihydroxypropyl)-amid

Analog Beispiel 1 aus (5E)-(16RS)-16,19-Dimethyl-18,19-didehydro-6a-carbaprostaglandin-$I_2$-11,15-bis-tetrahydropyranyläther und 1-Amino-2,3-dihydroxypropan.

IR ($CHCl_3$): 3600, 3390 (br.), 2915, 1690, 1580, 965/cm

**Patentansprüche**

1. Carbacyclinamide der Formel I

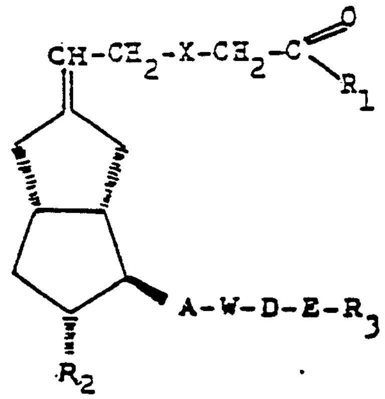

(I),

worin

$R_1$ die Reste $NHR_4$ mit $R_4$ als niederer Mono- oder Polyhydroxyalkylgruppe mit 2 - 8 C-Atomen,
$R_2$ eine Hydroxygruppe,
$R_3$ eine Alkylgruppe mit 1 - 7 C-Atomen,
X die Gruppe $-CH_2-$,
A eine trans-CH=CH- oder -C≡C-Gruppe,

W eine Hydroxymethylengruppe oder eine 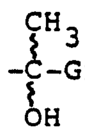 -Gruppe,

wobei die OH-Gruppe α- oder β-ständig sein kann,

D die Gruppe 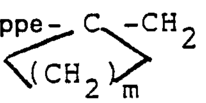 mit m gleich 1 - 3, eine geradkettige Alkylengruppe mit 1 - 5 C-Atomen oder eine verzweigte Alkylengruppe mit 2 - 5 C-Atomen,
E eine -C≡C-Gruppe oder eine $-CR_6=CR_7$-Gruppe, wobei $R_6$ und $R_7$ ein Wasserstoffatom oder eine Alkylgruppe mit 1 - 5 C-Atomen bedeuten, darstellen.

2. Verfahren zur Herstellung von Carbacyclinamiden der Formel I, dadurch gekennzeichnet, daß man eine Verbindung der Formel II

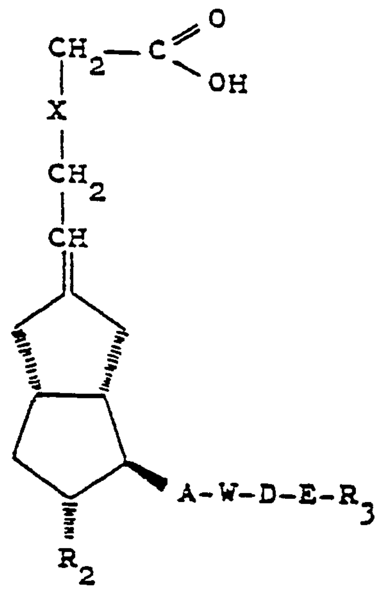

(II),

worin

$R_2$, $R_3$, X, A, W, D und E die oben angegebenen Bedeutungen aufweisen, gegebenenfalls nach Schutz anwesender freier Hydroxygruppen mit Aminen der Formel III

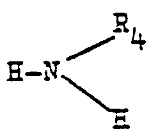

(III),

worin $R_4$ die oben angegebene Bedeutung aufweist, nach vorheriger Umsetzung mit Chlorameisensäureisobutylester in Gegenwart eines tertiären Amins umsetzt und gegebenenfalls anschließend geschützte Hydroxygruppen freisetzt und freie Hydroxygruppen verestert oder veräthert und in die Isomeren trennt.

3. Arzneimitel, bestehend aus einer oder mehreren Verbindungen des Anspruches 1 und üblichen Hilfs- und Trägerstoffen

4. (5E)-(16RS)-16-Methyl-18,18,19,19-tetradehydro-6a-carbaprostaglandin-$I_2$-(2,3-dihydroxy-propyl)-amid

5. (5E)-(16RS)-16-Methyl-18,18,19,19-tetradehydro-6a-carbaprostaglandin-$I_2$-(2-hydroxy-äthyl)-amid

## Claims

1. Carbacyclin amides of the formula I

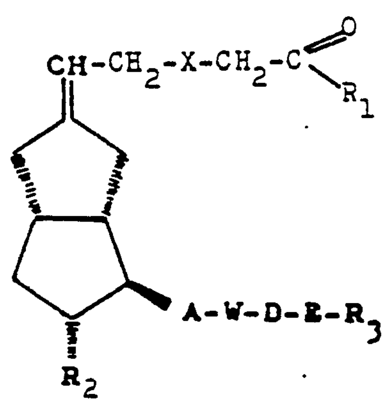

(I)

wherein

$R_1$ represents the radical $NHR_4$ wherein $R_4$ represents a mono- or poly-hydroxy-lower alkyl group having from 2 to 8 carbon atoms,

$R_2$ represents a hydroxy group,
$R_3$ represents an alkyl group having from 1 to 7 carbon atoms,
X represents the group $-CH_2-$,
A represents a <u>trans</u>-$CH=CH-$ or $-C\equiv C-$ group,

W represents a hydroxymethylene group or a 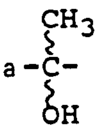 group wherein the OH group may be in the α- or β-configuration,

D represents the group 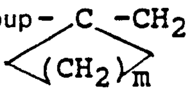 wherein <u>m</u> represents from 1 to 3, a straight-chain alkylene group having from 1 to 5 carbon atoms or a branched alkylene group having from 2 to 5 carbon atoms, and

E represents a $-C\equiv C-$ group or a $-CR_6=CR_7-$ group wherein $R_6$ and $R_7$ represent a hydrogen atom or an alkyl group having from 1 to 5 carbon atoms.

2. Process for the preparation of carbacyclin amides of the formula I, characterised in that a compound of the formula II

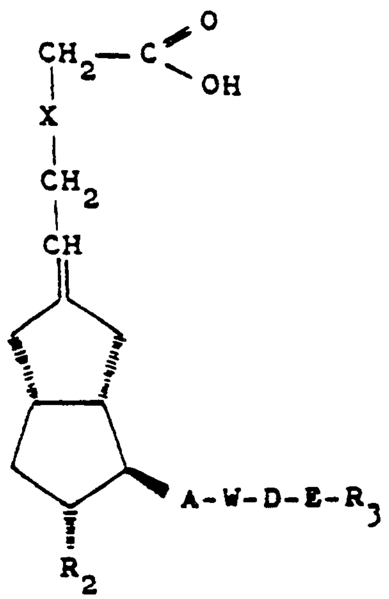

(II)

wherein

$R_2$, $R_3$, X, A, W, D and E have the meanings given above, is reacted, optionally, after protecting free hydroxy groups present, with amines of the formula III

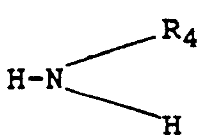

(III)

wherein $R_4$ has the meaning given above, after previous reaction with chloroformic acid isobutyl ester in the presence of a tertiary amine, and then, optionally, protected hydroxy groups are freed and free hydroxy groups are esterified or etherified and separated into the isomers.

3. Medicament, comprising one or more compounds of claim 1 and customary adjuncts and carriers.

4. (5<u>E</u>)-(16<u>RS</u>)-16-methyl-18,18,19,19-tetradehydro-6a-carbaprostaglandin-$I_2$ (2,3-dihydroxypropyl)-amide.

5. (5<u>E</u>)-(16<u>RS</u>)-16-methyl-18,18,19,19-tetradehydro-6a-carbaprostaglandin-$I_2$-(2-hydroxyethyl)-amide.

**Revendications**

1. Carbacyclinamides répondant à la formule I:

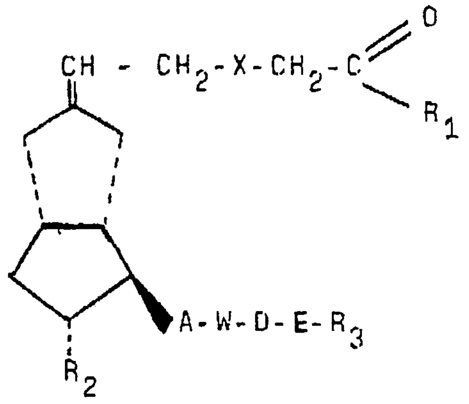

(I),

dans laquelle

- $R_1$ représente un radical $NHR_4$ dont le symbole $R_4$ désigne un radical mono- ou poly-hydroxyalkyle inférieur contenant de 2 à 8 atomes de carbone,
- $R_2$ représente un radical hydroxy,
- $R_3$ représente un radical alkyle en $C_1$ - $C_7$,
- X représente un radical $-CH_2-$,
- A représente un radical $-CH{=}CH-$ trans ou un radical $-C{\equiv}C-$,
- W représente un radical hydroxyméthylène ou un radical

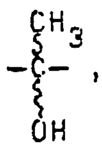

, dont le radical -OH peut avoir la configuration α ou la configuration β,

- D représente un radical

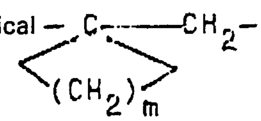

dont l'indice m désigne un nombre de 1 à 3, ou représente un radical alkylène linéaire contenant de 1 à 5 atomes de carbone ou un radical alkylène ramifié contenant de 2 à 5 atomes de carbone,
- E représente un radical $-C{\equiv}C-$ ou un radical $-CR_6{=}CR_7-$ dont les symboles $R_6$ et $R_7$ représentent chacun un atome d'hydrogène ou un radical alkyle en $C_1$ - $C_5$.

2. Procédé pour préparer des carbacyclinamides de formule (I), procédé caractérisé en ce qu'on fait réagir un composé répondant à la formule II

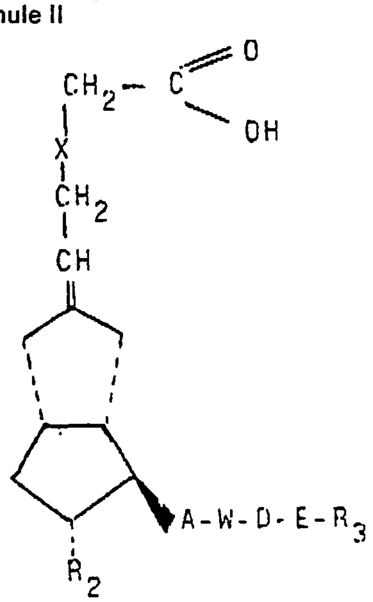

(II)

dans laquelle $R_2$, $R_3$, X, A, W, D et E ont les significations qui leur ont été données ci-dessus, après en avoir éventuellement protégé des radicaux hydroxy libres et après réaction avec le chloroformiate d'isobutyle en présence d'une amine tertiaire, avec une amine répondant à la formule III:

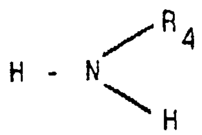

(III)

dans laquelle $R_4$ a la signification qui lui a été donnée ci-dessus, puis, le cas échéant, on libère des radicaux hydroxy protégés et on estérifie ou éthérifie des radicaux hydroxy libres, et on sépare les isomères.

3. Médicament constitué d'un ou de plusieurs composés selon la revendication 1 et d'excipients et adjuvants usuels.

4. (Dihydroxy-2,3 propyl)-amide de la méthyl-16 tétradéhydro-18,18,19,19 carba-6a prostaglandin $I_2$ (5E)-(16RS).

5. (Hydroxy-2 éthyl)-amide de la méthyl-16 tétradéhydro-18,18,19,19 carba-6a prostaglandin $I_2$ (5E)-(16RS).